# EUROPEAN PATENT APPLICATION

(11) **EP 3 398 617 A1**
(43) Date of publication of application: **07.11.2018**
(21) Application number: 17169483.9
(22) Date of filing: 04.05.2017
(51) Int. Cl.: A61L 2/04, B08B 3/02, B08B 3/08, B08B 3/10, A47L 15/00, A61L 2/18, B05B 3/14

(54) **A METHOD FOR WASHING AND DISINFECTING OBJECTS**

(71) Applicant: Getinge Disinfection AB, 351 15 Växjö (SE)
(72) Inventor: Samuelsson, Marcus, 352 63 VÄXJÖ (SE); Holm, Mats, 352 61 VÄXJÖ (SE)
(74) Representative: AWA Sweden AB

(57) **Abstract**

The present invention relates to a method for washing and disinfecting objects in awasher-disinfector comprising at least one chamber. The method comprises the steps of washing the objects in the chamber by circulating water and detergent in the chamber, rinsing the objects in the chamber by spraying non-circulating pressurized water in the chamber, draining the chamber, and disinfecting the objects. The steps of rinsing the objects and draining the chamber are performed at least partially simultaneously. The present invention allows for reduced water and energy consumption compared to prior art, while at the same time decreasing the risk for transfer of process residuals compared to when no rinsing is performed.

## Description

### Field of the invention

The present invention relates to a method for washing and disinfecting objects in a washer-disinfector.

### Background

Washer-disinfectors are intended for cleaning and disinfecting reusable objects used in fields such as medicine and dentistry. Due to the high demands that exist on cleanliness in such environments, there are EN/ISO-standards describing requirements for washer-disinfectors (ISO 158833 part 1-7).

There are different types of washer-disinfectors, one of which is called cart washer-disinfector. This type of device typically comprises a single chamber having a plurality of oscillating nozzles arranged inside the chamber, for circulating water. Transport trolleys, wash carts holding various types of objects, or other bulky items are typically load for a cart washer.

Another type of washer-disinfector is called instrument washer-disinfectors. These are smaller than cart washer-disinfectors, but the process typically performed by the different types of washer-disinfectors is the same. The process normally comprises several phases; pre-rinse, wash, post-rinse, final rinse/disinfection, and drying. All of the steps of washing and rinsing the objects involve circulating water inside the chamber using the oscillating nozzles. For the steps of rinsing the objects, only water is used. During the step of washing the objects, a detergent is added to the water. The final rinse/disinfection typically comprises circulating water of high temperature (approximately 90°C for thermal disinfection) inside the chamber. Chemical disinfection is also an option.

A cart washer-disinfector requires a large amount of water, typically about 150 litres, for one rinse or wash. Therefore, in particular for cart washer-disinfectors, to save time and reduce the total water consumption, pre-rinse and post-rinse are not always included in the process. When these steps are omitted, the risk for transfer of process residuals between phases increases. Process residuals may for example be detergent or soil. If residuals of a detergent are transferred to the phase in which the objects are disinfected, this may damage the coating of the chamber or the objects being washed, due to the high water temperature used during thermal disinfection or at a next steam sterilization step. Residual concerns also apply for the chemical disinfection process, which normally takes place at 60°C.

### Summary of the invention

It is an object of the present invention to alleviate at least some of the mentioned drawbacks of the prior art, and to provide an improved method for washing and disinfecting objects that reduces the transfer of residuals between phases in a more time and energy efficient manner.

The present invention is based on the realisation that if the objects are rinsed by spraying them with non-circulating pressurized water after they have been washed, instead of being rinsed by circulating water in the chamber containing the objects, the water and energy consumption can be reduced. At the same time, the risk for transfer of process residuals can be decreased compared to when no rinsing is performed.

When circulating water, the chamber needs to be filled to a certain level to allow the circulation pump to prime and pressurize the system. Using a separate pump system allows less water to be used when rinsing comprises spraying non-circulating pressurized water instead of circulating water as in the prior art.

According to a first aspect of the present invention, a method is provided for washing and disinfecting objects in a washer-disinfector comprising at least one chamber. Said method comprises the steps of: washing said objects in said chamber by circulating water and detergent in said chamber; rinsing the objects in said chamber by spraying non-circulating pressurized water in said chamber; draining said chamber; and disinfecting said objects. The steps of rinsing the objects and draining said chamber are performed at least partially simultaneously.

"At least partially simultaneously" can include at least partly simultaneously.

The simultaneous rinsing of the objects and draining of the chamber is possible since the water used for rinsing is not circulated in the chamber. This results in that the step of rinsing takes little or no additional time compared to if the step of rinsing would have been omitted.

The reduced risk of transfer of residuals compared to when no rinsing is performed can be verified by measuring the conductivity in the waste water. Presence of a detergent increases the conductivity of the water, so by comparing the conductivity when the objects are rinsed using non-circulating pressurized water with the conductivity when no rinsing is performed, the reduction of residuals can be verified.

According to at least one exemplary embodiment, the step of rinsing the objects by spraying non-circulating pressurized water over them comprises spraying 10-30 litres of water. This embodiment may suitably be implemented in a cart washer-disinfector. As described in relation to prior art, a traditional rinse in a cart washer-disinfector, using circulating water, uses about 150 litres of waters. A rinse according to an exemplary embodiment of the present invention hence uses approximately 7-20% of the volume of water used for a rinse according to prior art.

According to at least one exemplary embodiment of the present invention, the step of disinfecting said objects is performed in said chamber. By performing all steps of the method in a single chamber, the size of the washer-disinfector can be limited, and there is no need for transferring the objects between chambers.

According to at least one exemplary embodiment, the step of disinfecting said objects comprises using thermal disinfection. This is preferred since it is an efficient way of disinfecting without need for additional components such as chemicals.

According to at least one exemplary embodiment, the step of disinfecting said objects comprises circulating water in said chamber. Said water has a temperature of 80-95°C for thermal disinfection. These temperatures are suitable for achieving thermal disinfection.

According to at least one exemplary embodiment, said method further comprises a second step of draining said chamber. Said second step of draining may be performed after the step of disinfecting said objects. This is preferred when the step of disinfecting the objects comprises circulating water in said chamber.

According to at least one exemplary embodiment, said non-circulating pressurized water used for rinsing the objects has substantially the same temperature as the water used for disinfecting said objects. Substantially may mean that the difference in temperature between the two waters is no more than ±5°C. This is preferred since using water of a temperature lower than the water used for disinfection would result in the objects being cooled down before disinfection, and it would then take longer time for them to reach the required temperature during disinfection. Using water of substantially the same temperature hence contributes to limiting the duration of the process.

According to at least one exemplary embodiment, said water used for rinsing the objects and the water used for disinfecting said objects are supplied from a common water tank. This is advantageous since it is a simple way to ensure that the water used for rinsing and the water used for disinfecting have the same temperature. It is also preferred since it limits the number of water sources that are needed. According to at least one exemplary embodiment, the steps of rinsing and disinfecting the objects may use different sets of nozzles. This is advantageous since a higher flow rate might be desired for the step of disinfecting the objects, when this step comprises circulating water in the chamber. According to another exemplary embodiment, the steps of rinsing and disinfecting the objects may use the same set of nozzles.

According to at least one exemplary embodiment, said water used for washing said objects is supplied from a water source separate from said common water tank. Said water source could for example be a separate water tank or a water supply network. This is preferred since the water used for washing should not have a temperature as high as the temperature used for disinfecting, since using a detergent with water of such a high temperature can cause damage to the inside of the chamber of the washer-disinfector, or to the objects contained inside it.

According to at least another exemplary embodiment, said water used for rinsing the objects and the water used for disinfecting said objects are supplied from two different water tanks, or from two different water sources. This may be preferred since the water used for disinfection needs to be purified while regular water could be used for rinsing.

According to at least another exemplary embodiment, said step of disinfecting said objects comprises using chemical disinfection. According to at least one exemplary embodiment, the step of disinfecting said objects using chemical disinfection comprises using water of a temperature between 50-60°C, depending on the type of disinfectant used. This is advantageous since a lower temperature is needed for chemical disinfection compared to thermal disinfection, and chemical disinfection can therefore be used for objects that would be damaged from a temperature as high as that used for thermal disinfection.

According to at least one exemplary embodiment, said step of rinsing the objects comprises spraying said non-circulating pressurized water using a set of oscillating nozzles. According to another exemplary embodiment, said set of oscillating nozzles is a first set, and the step of washing said objects in said chamber comprises using a second set of oscillating nozzles for distributing the circulating water. This is advantageous since different types of nozzles may be needed for achieving the desired distribution of the water. According to at least one exemplary embodiment, the step of disinfecting the objects comprises using the second set of oscillating nozzles for distributing the circulating water. According to at least another exemplary embodiment, the step of disinfecting the objects comprises using the first set of oscillating nozzles for distributing the circulating water.

According to at least one exemplary embodiment, the oscillation of said first and second set of nozzles is synchronised. This allows the nozzles to be mounted on a common oscillation means.

According to at least another exemplary embodiment, said method further comprises a step of drying said objects, said step being performed after the step of disinfecting said objects. According to one exemplary embodiment, the drying of said objects is performed in said chamber. This is preferred since it is time efficient to dry the objects in the same chamber as they have been washed, rinsed and disinfected in.

According to at least one exemplary embodiment, said method further comprises one or more additional steps of rinsing said objects in said chamber by spraying non-circulating pressurized water in said chamber. This additional step or steps can be performed after the step of disinfecting said objects, and/or before the step of washing said objects, to further reduce the transfer of residuals.

According to at least one exemplary embodiment, said method further comprises collecting at least a part of the water used for rinsing the objects and reusing said water in the step of washing said objects in a later repetition of said method. This is advantageous since this further lowers the total water consumption.

According to at least one exemplary embodiment, said washer-disinfector is a cart washer-disinfector. According to another exemplary embodiment, said washer-disinfector is an instrument washer-disinfector.

According to a second aspect of the present invention, a washer-disinfector adapted for performing a method according to the present invention is provided. The washer-disinfector comprises a chamber, a first set of oscillating nozzles arranged in said chamber, and a second set of oscillating nozzles arranged in said chamber. Said first set of oscillating nozzles is connected to a first water source, and a pump is arranged to pressurize the water supplied from said first water source to the first set of oscillating nozzles. The second set of oscillating nozzles are arranged to enable circulation of water, from said first water source and/or from a second water source, in said chamber.

The pump and first set of nozzles may be adapted to be used for rinsing the objects in the chamber by spraying non-circulating pressurized water over them. The water may be taken from the first water source. The second set of nozzles may be adapted to be used for circulating water in said chamber, as is done when the objects are being washed and disinfected. When the objects are washed, water is suitably taken from the second water source. During disinfection, water is suitably taken from the first water source.

According to at least one exemplary embodiment of a washer-disinfector according to the present invention, the first water source is a water tank. This is advantageous since the quality of the water can be easily controlled in a tank, which is beneficial since the first water source may suitably be used for disinfection.

According to at least another exemplary embodiment, said water tank is preheated. This is advantageous since this enables heating the water to the desired temperature for disinfection before it reaches the chamber.

According to at least another exemplary embodiment, the temperature of the water in said preheated water tank is adjustable. This is advantageous since different water temperatures may be needed for different types of objects.

According to at least one exemplary embodiment, the oscillation of said first and second set of nozzles is synchronised. This is advantageous since a common oscillation means can be used for both sets of nozzles.

According to at least one exemplary embodiment, the first set of nozzles comprises orifices that are relatively small compared to orifices comprised in the second set of nozzles. This is advantageous since larger orifices allows for a higher flow rate, which is desired when a large volume of water is to be circulated.

According to a third aspect of the present invention a washer-disinfector adapted for performing a method according to the present invention is provided. The washer-disinfector comprises at least one chamber, means for washing said objects in said chamber by circulating water and detergent in said chamber, means for rinsing the objects in said chamber by spraying non-circulating pressurized water in said chamber, means for draining said chamber, and means for disinfecting said objects, wherein the washer-disinfector is configured so that rinsing the objects by spraying non-circulating pressurized water in said chamber and draining said chamber may be performed at least partially simultaneously.

A washer-disinfector according to the second and/or third aspect of the present invention may be used for performing a method according to the first aspect of the present invention.

Further objects, features and advantages of the present invention will become apparent from the following detailed description, the drawings and the appended claims.

### Brief description of the drawings

For exemplifying purposes, the invention will be described in closer detail in the following with reference to the appended drawings showing at least five different exemplary embodiments of the invention.
Figure 1 is a flowchart of a first exemplary embodiment of a method according to the first aspect of the present invention.
Figure 2 is a flowchart of a second exemplary embodiment of a method according to the first aspect of the present invention.
Figure 3 is a flowchart of a third exemplary embodiment of a method according to the first aspect of the present invention.
Figure 4 is a flowchart of a fourth exemplary embodiment of a method according to the first aspect of the present invention.
Figure 5 is a schematic illustration of an exemplary embodiment of a washer-disinfector according to the second and/or third aspect of the present invention, adapted for performing a method according to the first aspect of the present invention.

### Detailed description of embodiments

In the following detailed description exemplary embodiments of the present invention will be described. However, it is to be understood that features of the different embodiments are exchangeable between the embodiments and may be combined in different ways.

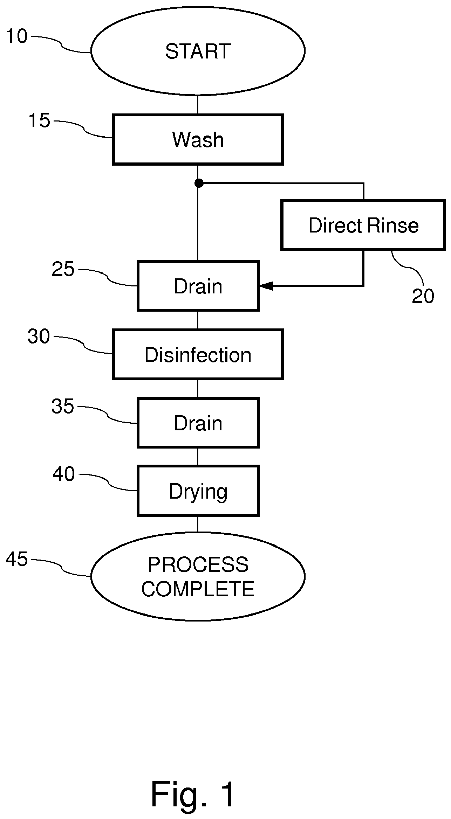

Figure 1 shows a flowchart of a method of washing and disinfecting objects in a washer disinfector, according to at least a first exemplary embodiment of the first aspect of the present invention. After start (e.g., step 10), the first step of said first exemplary embodiment is the step of washing the objects (e.g., step 15). During this step, water and a detergent is circulated in a chamber of the washer-disinfector. A set of oscillating nozzles is used for the circulation. When the process is performed in a cart washer-disinfector, this step uses approximately 150 litres of water. This water can be taken directly from a water supply network. Alternatively, it may be taken from a water tank. In some embodiments, the water used for washing may be mixed with waste water from a previous iteration of the method.

After the wash, two steps are performed in parallel: drain (e.g., step 25) and "Direct Rinse" (e.g., step 20). The "Direct Rinse"-step comprises rinsing the objects in said chamber by spraying non-circulating pressurized water over them. In this embodiment, "Direct Rinse" is performed using a set of nozzles other than the set used for the step of washing the objects. The set of nozzles used for "Direct Rinse" may have smaller orifices, and hence give a lower flow rate, than the set of nozzles used for washing the objects. The water for "Direct Rinse" is in this first embodiment taken from a pre-heated water tank. A pump is used to supply the water to the nozzles and to achieve a pressurization. The temperature of the water in the water tank can be adjusted. In this first exemplary embodiment, the temperature of the water in the water tank may be 90°C.

During the step of draining, the chamber is emptied of the water used for the wash as well as the water used for the "Direct Rinse". The drain- and "Direct Rinse"-steps are allowed to be performed simultaneously since the water used for rinsing in "Direct Rinse" is not circulated in the chamber.

When the method according to the exemplary embodiment is performed in a cart washer-disinfector, the "Direct Rinse"-step uses 10-30 litres of water.

After the "Direct Rinse" and draining of the chamber have been performed, a disinfection (e.g., step 30) is performed. In this first exemplary embodiment, this step comprises circulating water with a temperature of 90°C in said chamber. The water is taken from the same water tank as the water used for the "Direct Rinse", but for the step of disinfecting the objects the water is supplied through the same set of nozzles as is used for the step of washing the objects. This is advantageous since the larger orifices comprised by this set of nozzles are more efficient when circulating water, since they provide a higher flow rate.

After the objects have been disinfected, this first exemplary embodiment comprises another step of draining the chamber (e.g., step 35), to remove the water used for the step of disinfecting the objects. After this a final step of drying the objects is performed (e.g., step 40). In this embodiment, drying is achieved by heating the air inside the chamber. After drying, the process is complete (e.g. step 45).

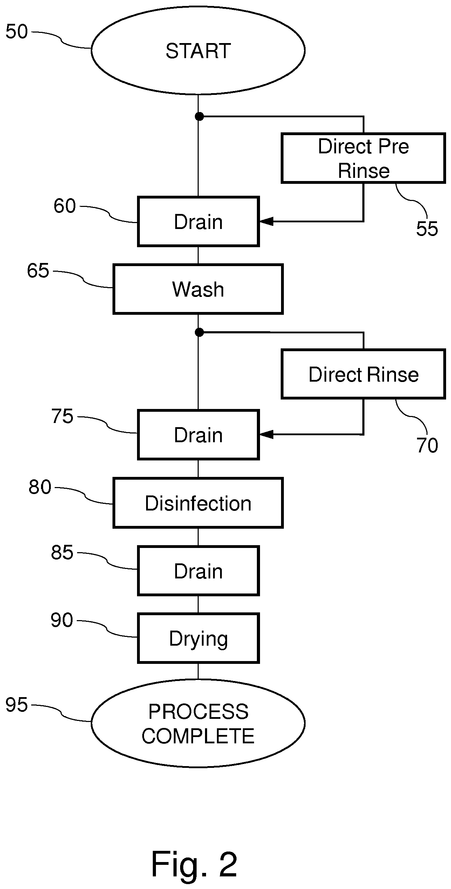

Figure 2 shows a flowchart of a method according to a second exemplary embodiment of the first aspect of the present invention. This embodiment comprises the steps of start (e.g., step 50), washing (e.g., step 65), rinsing using "Direct Rinse" (e.g., step 70), draining (e.g., step 75), disinfecting (e.g., step 80), draining (e.g., step 85) and drying the objects (e.g., step 90) in accordance with the first exemplary embodiment. After drying, the process is complete (e.g., step 95). The method according to this second embodiment further comprises a step called "Direct Pre Rinse" (e.g., step 55), performed at the beginning of the process. This step is performed in the same way as the step "Direct Rinse". Non-circulating pressurized water is sprayed over the objects in said chamber. In this embodiment, this is done using the same set of nozzles as is used for the "Direct Rinse". The water may be taken from the same water tank as for "Direct Rinse" and disinfection. Alternatively, the water may be taken from the water supply network or from a separate water tank. If the soil on the objects contains proteins, for example blood, it is advantageous to use cold water for the "Direct Pre Rinse". "Direct Pre Rinse" is followed by an additional step of draining said chamber (e.g., step 60).

The purpose of adding a step of "Direct Pre Rinse" may be to further reduce transfer of residuals, in this case residuals being soil, and to efficiently rinse the objects using a limited amount of water before washing them.

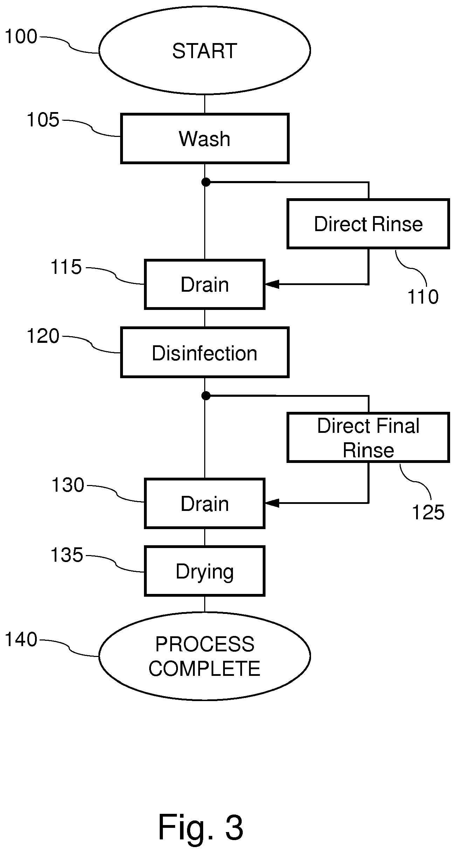

Figure 3 shows a flowchart of a method according to a third exemplary embodiment of the first aspect of the present invention. This embodiment comprises the steps of start (e.g., step 100), washing (e.g., step 105), rinsing using "Direct Rinse" (e.g., step 110), draining (e.g., step 115), disinfecting (e.g., step 120), draining (e.g., step 130) and drying the objects (e.g., step 135) in accordance with the first exemplary embodiment. After drying, the process is complete (e.g., step 140) The method according to this third embodiment further comprises a step called "Direct Final Rinse" (e.g., step 125), performed after the step of disinfecting the objects. This is a repetition of the "Direct Rinse"-step. Non-circulating pressurized water is sprayed over the objects in said chamber. This may be done using the same set of oscillating nozzles as for "Direct Rinse". Alternatively, the same set of oscillating nozzles as are used for washing may be used. The water used for "Direct Final Rinse" may be taken from the same water tank that supplies water for disinfection and "Direct Rinse". Alternatively, the water may be taken from the water supply network, or from a separate water tank.

The purpose of adding a step of "Direct Final Rinse" may be to further reduce process residuals on the objects, especially if the high quality of the water used for disinfection is limited.

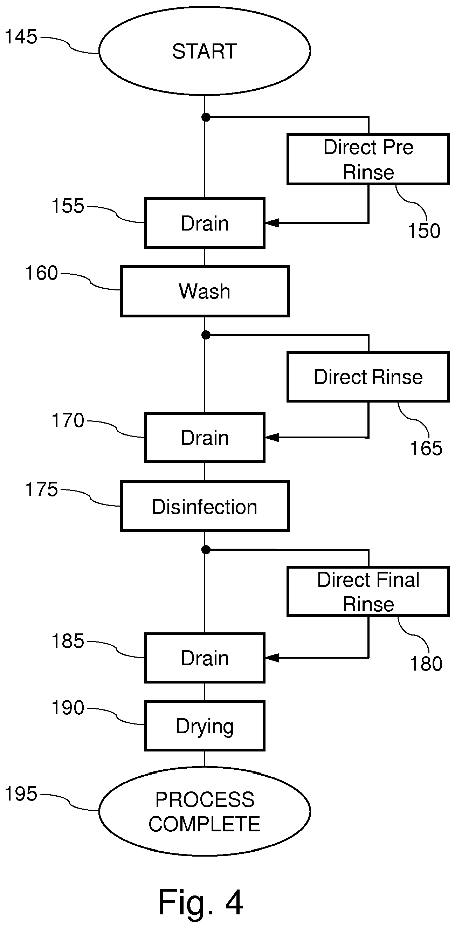

Figure 4 shows a flowchart of a method according to a fourth exemplary embodiment of the first aspect of the present invention. This embodiment is a combination of the first, second and third embodiments; it comprises the steps of start (e.g., step 145), washing (e.g., step 160), rinsing using "Direct Rinse" (e.g., step 165), draining (e.g., step 170), disinfecting (e.g., step 175), draining (e.g., step 185) and drying the objects (e.g., step 190) in accordance with the first exemplary embodiment. After drying, the process is complete (e.g., step 195). The fourth embodiment further comprises the "Direct Pre Rinse" (e.g., step 150) and additional draining (e.g., step 155) in accordance with the second exemplary embodiment, as well as the "Direct Final Rinse" (e.g., step 180) in accordance with the third exemplary embodiment. The purpose of including both "Direct Pre Rinse" and "Direct Final Rinse", in addition to the step of "Direct Rinse", may be to further limit the transfer of process residuals between the phases of the process.

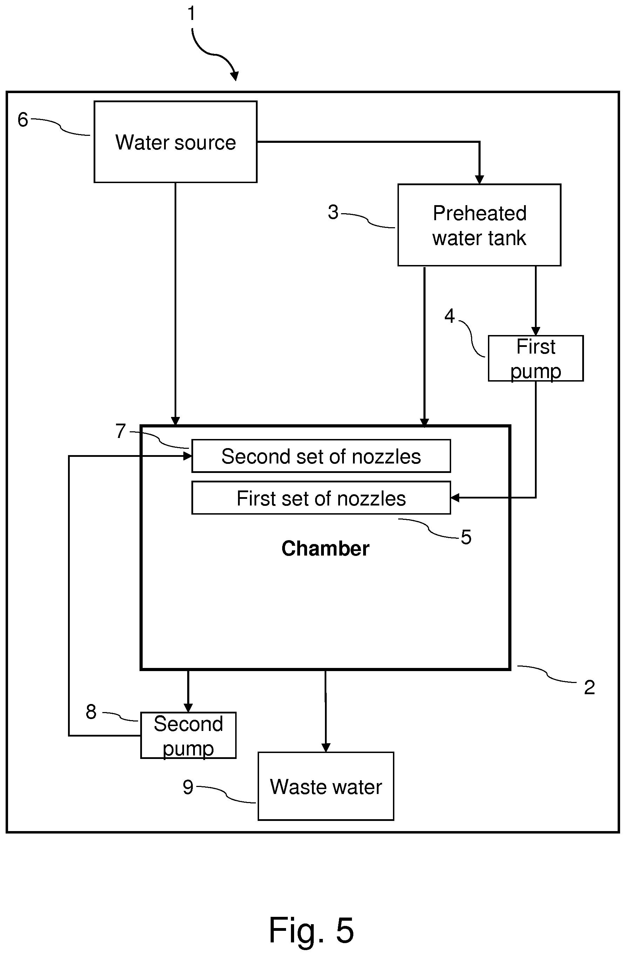

Figure 5 shows a schematic illustration of a washer-disinfector 1 according to an exemplary embodiment of the second and/or third aspect of the present invention. The washer-disinfector is adapted for performing a method according to the first aspect of the present invention. The washer-disinfector 1 comprises a chamber 2 where the objects may be placed, a preheated water tank 3, a first pump 4, a first set of nozzles 5, a water source 6, a second set of nozzles 7, a second pump 8, and an outlet for waste water 9. The way water may flow between the different parts of the machine is illustrated with arrows in the figure.

The washer-disinfector 1 may be a cart washer-disinfector or an instrument washer-disinfector. The water source 6, the second set of nozzles 7 and the second pump 8 may constitute or be comprised in means for washing objects in the chamber 2 by ci rculati ng water and detergent therein. The preheated water tank 3, the first pump 4 and the first set of nozzles may make up or be comprised in means for rinsing the objects in the chamber 2 by spraying non-circulating pressurized water therein. Means for draining the chamber 2 may comprise or be provided by the waste water outlet 9. The preheated water tank 3 together with the second set of nozzles 7 and the second pump 8 may provide or be comprised in means for disinfecting the objects. The washer-disinfector 1 is configured so that rinsing the objects by spraying non-circulating pressurized water in said chamber 2 and draining said chamber 2 may be performed at least partially simultaneously.

The water source 6 may be a water supply network or a water tank. The water source 6 may provide water directly to the chamber 2, and it may also optionally provide water to the preheated water tank 3. In an alternative embodiment, the preheated water tank 3 may be omitted, so that water is supplied directly from the water source 6 via the first pump 4 to the first set of nozzles 5.

The second set of nozzles 7 may comprise larger orifices than the first set of nozzles 5, allowing a higher flow rate. This is advantageous since the second set of nozzles 7 is adapted to be used for circulating a relatively large volume of water, while the first set of nozzles 5 is adapted for spraying non-circulating pressurized water.

Figure 5 is a schematic illustration of a washer-disinfector 1, hence not all components are included in the illustration. For example, conventional means for circulating and transporting water such as conduits may also be part of the washer-disinfector 1.

A method according to the first exemplary embodiment of the present invention, described in relation to Figure 1, may be explained further in relation to the washer-disinfector 1 illustrated in Figure 5. During the first step of the method, washing the objects 15, water is taken from the water source 6. The water is supplied to the chamber 2 holding the objects and circulated therein using the second set of nozzles 7 and the second pump 8. The water is circulated together with a detergent.

After the step of washing the objects, the chamber is drained 25. During this step, the circulated water and detergent is emptied out through the waste water outlet 9. During draining, the "Direct Rinse"-step 20 is also performed. Water is taken from the preheated water tank 3, which may be filled with water from the water source 6, and supplied to the first set of nozzles 5 using the first pump 4. From the first set of nozzles 5, the water is sprayed over the objects. The water used for "Direct Rinse" is not circulated in the chamber 2. This water is also drained through the waste water outlet 9, together with the wash water. The waste water may be collected and at least a part of it may be reused in a later repetition of the method.

After the "Direct Rinse" and draining have been completed, the disinfection 30 is performed. During this step, water is taken from the preheated water tank 3 and circulated in the chamber 2 using the second set of nozzles 7, i.e. the same set of nozzles as were used for the step of washing the objects, and the second pump 8. After the disinfection is completed, the water in the chamber is drained 35 through the waste water outlet 9. This water may also be collected and at least a part of it may be reused in a later repetition of the method. Finally, the objects in the chamber are dried 40 by heating the air inside the chamber.

The washer-disinfector 1 described in relation to Figure 5 may also be used to perform any of the other exemplary embodiments of the invention.

The person skilled in the art realizes that the present invention by no means is limited to the embodiments described above. Different features of the present invention may be combined to create embodiments other than those described, and many modifications and variations are possible within the scope of the appended claims. For example, chemical disinfection may be used instead of thermal disinfection, and the method may be applied to instrument washer-disinfectors instead of cart washer-disinfectors. The steps of the method may be performed in any appropriate order, and the method is not limited to the order of the steps described herein.

## Claims

1. A method for washing and disinfecting objects in a washer-disinfector (1) comprising at least one chamber (2), said method comprising the steps of:
washing said objects in said chamber by circulating water and detergent in said chamber;
rinsing the objects in said chamber by spraying non-circulating pressurized water in said chamber;
draining said chamber; and
disinfecting said objects,
wherein the steps of rinsing the objects and draining said chamber are performed at least partially simultaneously.

2. A method according to claim 1, wherein the step of disinfecting said objects is performed in said chamber (2).

3. A method according to claim 2, wherein said step of disinfecting said objects comprises using thermal disinfection.

4. A method according to claim 3, wherein the step of disinfecting said objects comprises circulating water in said chamber (2), said water having a temperature of 80-95°C.

5. A method according to claim 4, wherein said method further comprises a second step of draining said chamber (2), said second step of draining being performed after the step of disinfecting said objects.

6. A method according to claim 4 or 5, wherein said non-circulating pressurized water used for rinsing the objects has substantially the same temperature as the water used for disinfecting said objects.

7. A method according to any one of claims 4-6, wherein said water used for rinsing the objects by spraying non-circulating pressurized water and the water used for disinfecting said objects are supplied from a common water tank (3).

8. A method according to claim 7, wherein said water used for washing said objects is supplied from a water source (6) separate from said common water tank.

9. A method according to claim 1 or 2, wherein said step of disinfecting said objects comprises using chemical disinfection, wherein a water temperature of 50-60°C is preferably used.

10. A method according to any one of the preceding claims, wherein said step of rinsing the objects comprises spraying said non-circulating pressurized water using a set of oscillating nozzles (5).

11. A method according to claim 10, wherein said set of oscillating nozzles (5) is a first set, and the step of washing said objects in said chamber (2) comprises using a second set of oscillating nozzles (7) for distributing the circulating water.

12. A method according to any one of the preceding claims, wherein said method further comprises a step of drying said objects, said step being performed after the step of disinfecting said objects.

13. A method according to any one of the preceding claims, further comprising one or more additional steps of rinsing said objects in said chamber (2) by spraying non-circulating pressurized water in said chamber (2).

14. A method according to any one of the preceding claims, further comprising collecting at least a part of the water used for rinsing the objects and reusing said water in the step of washing said objects in a later repetition of said method.

15. A method according to any one of the preceding claims, wherein said washer-disinfector (1) is a cart washer-disinfector.
